# NEW EUROPEAN PATENT SPECIFICATION

(11) **EP 0 291 986 B2**
(45) Date of publication and mention of the opposition decision: **21.01.1998**
(45) Mention of the grant of the patent: 06.10.1993
(21) Application number: 88108054.3
(22) Date of filing: 19.05.1988
(51) Int. Cl.: A61K 7/42

(54) **Oily sun-care cosmetic**
Ölartiges Sonnenschutz-Kosmetikum
Cosmétique huileux antisolaire

(30) Priority: 20.05.1987 JP 123350/87
(43) Date of publication of application: 23.11.1988
(73) Proprietor: SHISEIDO COMPANY LIMITED, Chuo-kuTokyo (JP)
(72) Inventor: Nakamura, Shin,c/o Shiseido Laboratories, Yokohama-shi,Kanagawa (JP); Takata, Sakaki,c/o Shiseido Laboratories, Yokohama-shi,Kanagawa (JP)
(74) Representative: Grünecker, Kinkeldey, Stockmair & SchwanhäusserAnwaltssozietät

(56) References cited:
- EP-A- 0 158 412
- DE-A- 2 014 152
- GB-A- 2 064 363
- US-A- 2 425 311
- US-A- 3 341 419
- US-A- 4 193 989
- US-A- 4 559 225
- US-A- 4 710 371
- US-A- 4 731 242
- HAPPI HOUSEHOLD & PERSONAL PRODUCTS INDUSTRY, vol. 17, no. 5, May 1980, Ramsey, NJ (US); H. BROWN, pp. 54, 56, 60, 62#
- Parfümerie und Kosmetik 67. Jahrgang, Nr. 6/86. pages 384-389
- G M Cameron et al, "Der Einsatz von flüchtigen Silikonen in Kosmetischen Anwendungen".

## Description

This invention relates to a suncare oil to be used in the sun in order to improve or prevent suntan.

A suntan oil is used in order to obtain beautifully and healthfully tanned skin of corn-color or bronze color while enjoying a sunbath. A conventional suntan oil mainly comprises liquid paraffin and/or vegetable oil(s) sometimes together with ester oil(s). Recently ultraviolet light absorber(s) are frequently added thereto to thereby protect the skin from toxic ultraviolet light.

For example, US-A-4193989 discloses a water resistant sun tan gel comprising an alcohol as volatile compound, cellulose and starch esters as gelling agents and, for example, silicone oil for flexibility and water resistance. Said esters and said silicone oil are no volatile compounds due to their high molecular weight.

GB-A-2064363 discloses stable silicone-water emulsions of volatile cyclic siloxanes which can be used for suntan lotions.

EP-A-158412 discloses a sunscreen composition consisting essentially of a film forming cellulosic polymer, a solvent for said film-forming cellulosic polymer, an ultraviolet absorbing sunscreening agent and an emollient. Alcohols are used as solvents for the film forming cellulosic polymers. Examples of the emollients are hydrocarbon oils and waxes, fatty acid esters and volatile silicone fluids.

Happi Household & Personal Products Industry, No. 17. No. 5, pages 54, 56, 60, 62, discloses sunscreen oils containing alcohols in different amounts.

Parfumerie und Kosmetik 67 Ed.,No. 6/86, pages 384-389 discloses compositions comprising cyclic dimethyl polysiloxanes of a polymerization degree of 5 and aluminum chlorohydrate. US-A-4 559 225 discloses a composition comprising volatile silicone fluids and cellulose ethyl ether.

However it is widely known that the application of a conventional suntan oil would sometimes cause unexpected uneven suntan. That is to say, some portions of the body are reddened or spotted after several days. Furthermore serious sunburn would be accompanied by partial blistering or peeling-off of the skin, which sometimes makes a person to shun the public. Thus some persons hesitate to obtain a good tan, although they would like to have beautifully and healthfully tanned skin.

The uneven suntan is caused by the unevenness in the thickness of the applied oil. Even though the oil is uniformly applied to the skin, it might subsequently flow thereon, thus causing uneven distribution. In particular, the thickness of the applied oil would become smaller on the protruding sites of the body such as shoulders, nose and cheekbones. Thus these sites are liable to be tanned. In addition, sand on the seashore might result in uneven suntan, since sand adhereing to the oil-coated skin prevent the skin from being tanned or the suntan oil might be brushed off together with the sand. The recent trend to add ultraviolet light absorber(s) to a suntan oil has further promoted the uneven suntan over the treated and untreated sites. Thus there are various causes for the uneven suntan and one sould take a great deal of care in order to obtain an even and beautiful tan.

As described above, an attempt to obtain a beautiful and healthy tan might frequently result in a failure.

It is, therefore, the object of the present invention to provide a suncare oil which enables an even and beautiful suntan and prevents sand-adhesion when applied to the skin.

According to the present invention this object is achieved by a suncare oil for preventing sand-adhesion when applied to skin, comprising at least 30% by weight of one or more volatile components selected from the group consisting of straight-chain, branched or cyclic and unsaturated hydrocarbons containing 4 to 12 carbon atoms, esters containing 4 to 8 carbon atoms in total, methylpolysiloxanes of a polymerization degree of 1 to 19 and cyclic dimethylpolysiloxanes of a polymerization degree of 2 to 7, with the proviso that the suncare oil does not contain aluminum hydrochloride or cellulose ethyl ether.

The volatile components to be used in the present invention are those which would volatilize when stored at 35°C under atmospheric pressure. Namely, 5.00 g of each component is stored in a container having a surface area of 19.625 cm² for one hour under the conditions as defined above and then weighed. When the component shows a weight loss, it is regarded as volatile.

The volatile components used according to the present invention are straight-chain, branched or cyclic and unsaturated hydrocarbons containing 4 to 12 carbon atoms, esters containing 4 to 8 carbon atoms in total and, as silicone materials, methylpolysiloxane of a polymerization degree of 1 to 19 and cyclic dimethylpolysiloxane of a polymerization degree of 2 to 7.

More particularly, hydrocarbon materials such as hexane, octane, decane, cyclohexane, isooctane, xylene or toluene; esters such as ethyl acetate or butyl acetate; and silicone materials such as methylpolysiloxane (polymerization degree of 5), decamethylcyclopentasiloxane or octamethylcyclotetrasiloxane are used.

The aimed product of the present invention is obtained by employing at least 30%, preferably at least 40% and still more preferably at least 60%, of one or more volatile components,

In addition to the volatile component(s), the oily sun-care cosmetic of the present invention may appropriately comprise various additives conventionally employed in the art, for example, mineral oil, vegetable oil, animal oil, higher alcohol, higher fatty acid, ester oil, nonvolatile silicone oil, ultraviolet light absorber, medicine, antioxidant, stabilizer, colorant, perfume and oil-soluble humectant depending on the aimed properties of the final product.

To further illustrate the present invention the following Examples and Comparative Example will be given, wherein all percentages are by weight.

### Example 1-5 and Comparative Examples 1-5

Each components shown in Table 1 were uniformly mixed with stirring at room temperature to obtain a final product.

The effects of the present invention are shown by the Examples and Comparative Examples as shown in Table 1. The effects of the present inveniton were evaluated by employing four young male subjects who were allowed to take free action for four hours on the seashore in midsummer. The color values were determined four days thereafter. Each test sample and the comparative sample were symmetrically applied to the shoulders, the center of the back and the waist of each subject. Each sample was applied again two hours after the first application. In each Example of an add number, the test sample was applied on the left side of the body while the comparative one was applied on the right side. On the other hand, in each Example of an even number, the test sample was applied on the right side of the body while the comparative one was applied on the left side, thus avoiding biased conditions.

The color values were determined by a color computer.

As shown in Table 1, the sand-adhesion, nonstickiness and refreshing feel of each product, which were reported by the subjects by themselves, were also evaluated in addition to the color value.

In every of the panels a to f, the brightness in the case of the samples of the present invention varied less around the body sites, as compared with the samples for comparison. These results indicate that the products of the present invention caused less unevenness than the comparative ones did. The incorporation of 30% or more of volatile components resulted in little variation in the brightness while the incorporation of less than 30% thereof resulted in significant variation (cf. panels e and f).

Thus it has been proved that the suncare oil of the present invention comprising at least 30% of the volatile component(s) as listed above enables an even and beautiful suntan. In addition, the oily sun-care cosmetic of the present invention comprising 40% or above of volatile component(s) would be accompanied by little sand-adhesion and have an unsticky refreshing and preferable feel at the use.

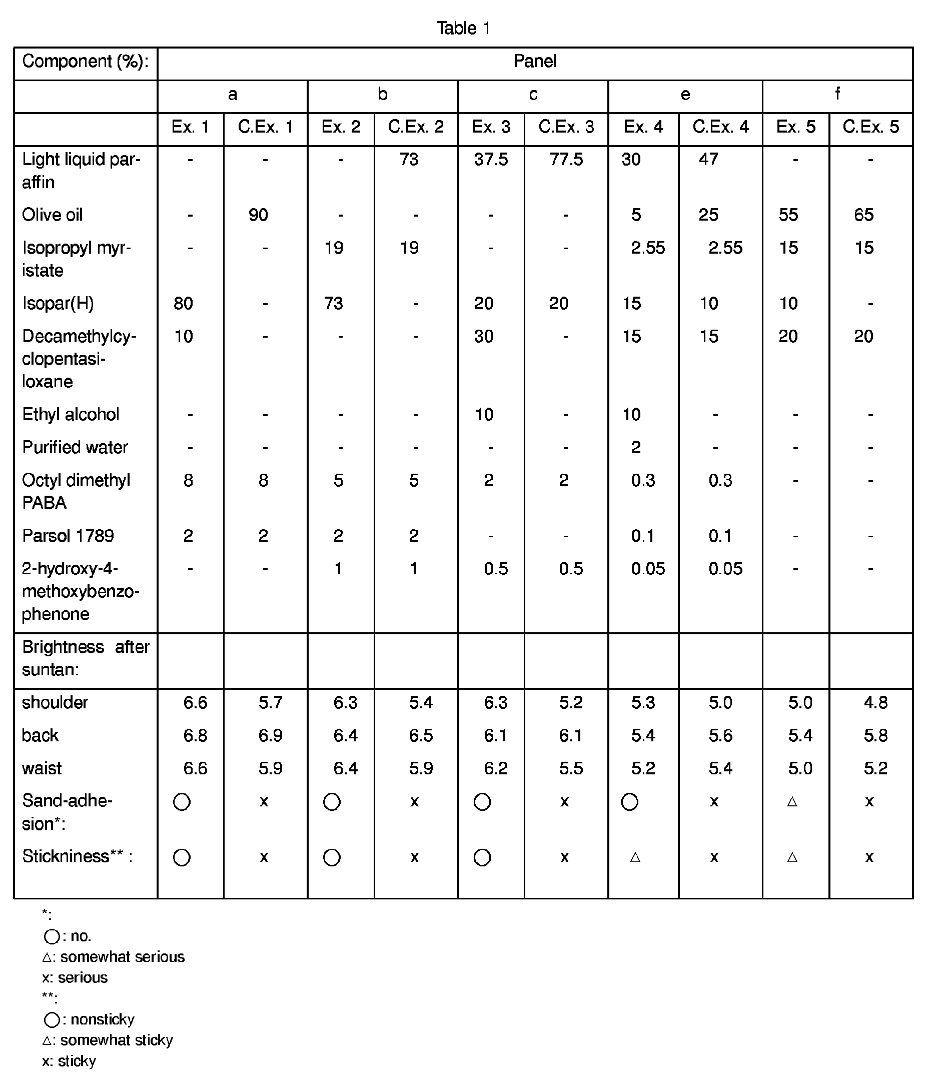

### Example 6

In the same manner as the preparation of Examples 1-5, each components shown below were uniformly mixed with stirring at room temperature to obtain a final product.

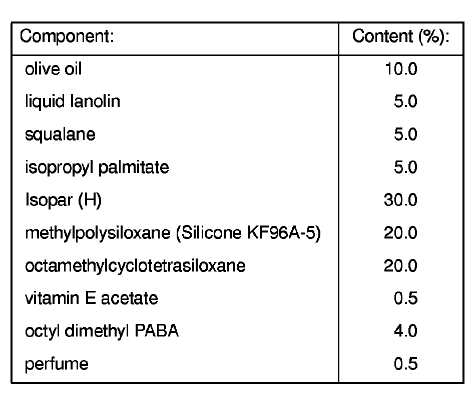

The obtained suncare oil would be accompanied by little sand-adhesion and have an unsticky, refrishing and preferable feel after the use.

After use of the suncare oil, there are no uneven suntan and inflammation.

### Example 7

In the same manner as the preparation of Examples 1-5, each components shown below were uniformly mixed with stirring at room temperature to obtain a final product.

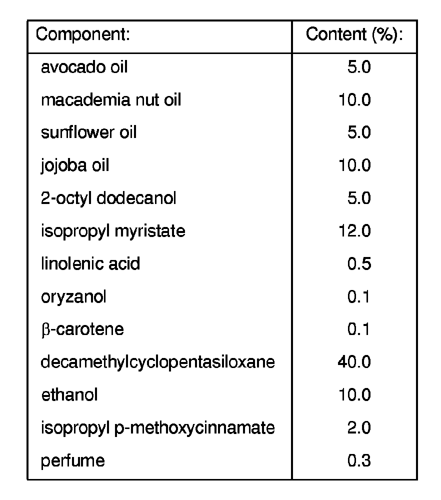

As in Example 6, the obtained suncare oil indicates an excellent usability and preferable suntan, thus this is an excellent suncare oil.

## Claims

1. A suncare oil for preventing sand-adhesion when applied to skin comprising at least 30% by weight of one or more volatile components selected from the group consisting of straight-chain, branched or cyclic and unsaturated hydrocarbons containing 4 to 12 carbon atoms, esters containing 4 to 8 carbon atoms in total, methylpolysiloxanes of a polymerization degree of 1 to 19 and cyclic dimethylpolysiloxanes of a polymerization degree of 2 to 7, with the proviso that the suncare oil does not contain aluminum hydrochloride or cellulose ethyl ether.

2. The suncare oil of claim 1, comprising at least 40% by weight of the one or more volatile components.

3. The suncare oil of claim 1, comprising at least 60% by weight of the one or more volatile components.

## Patentansprüche

1. Sonnenöl zum Verhindern der Anhaftung von Sand, wenn es auf die Haut aufgetragen ist, umfassend mindestens 30 Gew.-% von einer oder mehreren flüchtigen Komponenten, ausgewählt aus der Gruppe bestehend aus geradkettigen, verzweigten oder cyclischen und ungesättigten Kohlenwasserstoffen, die 4 bis 12 Kohlenstoffatome enthalten, Estern, die insgesamt 4 bis 8 Kohlenstoffatome enthalten, Methylpolysiloxanen mit einem Polymerisationsgrad von 1 bis 19 und cyclischen Dimethylpolysiloxanen mit einem Polymerisationsgrad von 2 bis 7, unter der Voraussetzung, daß das Sonnenöl kein Aluminiumhydrochlorid oder Celluloseethylether enthält.

2. Sonnenöl nach Anspruch 1, worin die flüchtigen Komponenten mindestens 40 Gew.-% betragen.

3. Sonnenöl nach Anspruch 1, worin die flüchtigen Komponenten mindestens 60 Gew.-% betragen.

## Revendications

1. Huile de protection solaire destinée à prévenir l'adhérence de sable lors de son application sur la peau, comprenant au moins 30 % en poids d'un ou plusieurs composants volatils sélectionnés dans le groupe constitué par des hydrocarbures linéaires, ramifiés ou cycliques et insaturés contenant 4 à 12 atomes de carbone, des esters contenant au total 4 à 8 atomes de carbone, des méthylpolysiloxanes ayant un degré de polymérisation compris entre 1 et 19 et des diméthylpolysiloxanes cycliques ayant un degré de polymérisation compris entre 2 et 7, pour autant que l'huile de protection solaire ne contient pas de chlorhydrate d'aluminium, ni d'éthyléther de cellulose.

2. Huile de protection solaire selon la revendication 1 comprenant au moins 40 % en poids d'un ou plusieurs composants volatils.

3. Huile de protection solaire selon la revendication 1 comprenant au moins 60 % en poids d'un ou plusieurs composants volatils.
